# EUROPEAN PATENT APPLICATION

(11) **EP 4 691 494 A1**
(43) Date of publication of application: **11.02.2026**
(21) Application number: 24306345.0
(22) Date of filing: 09.08.2024
(51) Int. Cl.: A61K 47/64, A61P 7/04

(54) **INHIBITORS OF ENDOTHELIAL APOPTOSIS FOR USE IN THE TREATMENT OF A CONDITION DUE TO MICROVASCULAR INJURY**

(71) Applicant: Centre National de la Recherche Scientifique, 75016 Paris (FR); Institut National de la Santé et de la Recherche Médicale, 75013 Paris (FR); Université de Montpellier, 34090 Montpellier (FR)
(72) Inventor: BARRERE-LEMAIRE, Stéphanie, 34680 Saint Georges d'Orques (FR); NARGEOT, Joël, 34090 Montpellier (FR); BOISGUERIN, Prisca, 34080 Montpellier (FR)
(74) Representative: Novagraaf Technologies

(57) **Abstract**

The invention relates to a conjugate comprising a peptide having the amino acid sequence set forth in SEQ ID NO: 1 or SEQ ID NO: 2, linked to a cell-penetrating peptide (CPP), for use in the treatment of a condition due to microvascular injury.

## Description

### Technical field

The present invention refers to inhibitors of endothelial apoptosis for use in the treatment of a condition due to microvascular injury.

Therefore, the present invention has utility in the medical field.

In the description below, the references in brackets ([ ]) refer to the listing of references situated at the end of the text.

### Background of the Invention

**Microhemorrhages** in various organs such as the heart or the brain have been associated with high mortality and devastating disabilities in patients. Apoptosis of endothelial cells has been reported to be a key event for microvascular injury leading to bleeding in organs upon viral infection, acute ischemia, and reperfusion injury.

The heart is a complex organ consisting of various cell types, each of which plays an important role in both physiological and pathophysiological conditions. Cardiomyocytes and endothelial cells are the two main and abundant cell types of the heart that play a key role in both cardiac remodeling and regeneration. In murine and human hearts, cardiomyocytes represent 30% of the cardiac cells and endothelial cells 50%. During pathological situations such as ischemia-reperfusion injury, these two cell types undergo death through oxidative stress, calcium overload, and inflammation.

Hemorrhages in the cardiac muscle are associated with severe ischemic injury during coronary occlusion. Microvascular damages and distal microthrombus embolization (microvascular obstruction) during the percutaneous coronary intervention (PCI) lead in up to 30% of patients to the no-reflow phenomenon, which is associated with larger infarct size and worse outcomes including a higher incidence of death. Furthermore, it has been clearly demonstrated that areas of microvascular obstruction and intramyocardial hemorrhage (IMH) overlap extensively, indicating that myocardial tissue exhibits vascular injury and extravasation. The leakage of circulating cells into the interstitium occurs also after the rupture of the vascular wall consequently in the massive swelling of capillary endothelial cells taking place in the coronary microvascular injury as a consequence of myocardial reperfusion (opening of the occluded coronary) and the abrupt restoration of the blood flow in coronary vessels.

Both no-reflow and IMH carry an adverse prognosis for patients with reperfused myocardial infarction. In particular, for STEMI (ST-segment elevation myocardial infarction) patients, microvascular injury occurs in up to 50% of patients and is associated with worse outcomes. Microvascular injury is a complex and heterogeneous phenomenon, mainly caused by severe ischemia/reperfusion (IR) injury together with distal embolization and subject-specific predispositions. Recent evidence suggests that IMH is not only an indicator of extensive myocardial infarction but also promotes infarct expansion after reperfusion and jeopardizes myocardial salvage. Moreover, it has been reported that IMH is the decisive factor for the prognosis of patients with microvascular injury after reperfusion for STEMI by contrast to microvascular obstruction (MVO) which is associated with a more favorable prognosis, rather similar to that without microvascular injury. This highlights the independent prognostic importance of IMH in assessing and managing risk after STEMI and more importantly the urgent need to develop a drug to treat intramyocardial hemorrhagic lesions.IR injury affects not only cardiomyocytes but also all other cellular compartments, including endothelial cells (ratio 3:1 endothelial *versus* cardiomyocytes within myocardial tissue), and coronary circulation has a central role in it. Microcirculatory injury is due to local accumulation of inflammatory cells after blood flow restoration, intracellular calcium overload, and the burst of reactive oxygen species (ROS) contributing to cell dysfunction and programmed cell death. Several studies have found that oxidative stress induces cellular death via both the mitochondrial-dependent and the death-receptor apoptotic pathways. In particular, our group has demonstrated the crucial role of the First Apoptosis Signal (FAS) death receptor downstream cascade in mediating IR-induced apoptosis (Roubille et al. 2007 ([1])). In addition, elevated Fas Ligand has been evidenced in the blood of AMI (Acute Myocardial Infarction) patients. Two adaptor proteins, FADD (Fas-Associated protein with Death Domain) and DAXX (death-associated protein) allow to coupling the extracellular signal to the intracellular apoptotic machinery. A high level of apoptosis has been described in injured vessels after angioplasty and the FAS-FADD dependent pathway in vascular cells contributes to both caspase-dependent apoptosis and pro-inflammatory gene induction via NF-kB. FADD protein expression was shown to be upregulated in the myocardium after IR and to play a critical role in post-ischemic remodeling and heart failure. More recently, FADD was identified as a biomarker of human coronary events.

Our group identified the FAS-DAXX dependent pathway as a key contributor to myocardial IR injury (Roubille et al. 2007 ([1])) since the proapoptotic function of DAXX was previously reported to be triggered by oxidative stress.

In the brain also, spontaneous intracerebral hemorrhage (SICH) is a subtype of stroke associated with high mortality and devastating disabilities. A study of patients with SICH found a higher content of FAS ligands in the perihematomic brain area compared to healthy brain areas. In hemorrhagic stroke (non-ischemic), the blood-brain barrier is disrupted, leading to an influx of water molecules and blood components into the brain's extracellular space, which results in vasogenic brain edema and hemorrhagic transformation as serious clinical consequences. Hemorrhagic stroke has both primary (physical disruption, increased intracranial pressure) and secondary (clot-derived cytotoxic factors, neuroinflammation, edema, secondary ischemia) injury components. In addition, hemorrhagic transformation is a major risk in post-ischemic stroke patients treated with thrombolytic therapy and thrombectomy. In vivo, studies in hemorrhagic stroke demonstrated that cell death in endothelial cells of microvessels was increased after subarachnoid hemorrhage as well as in the perihematomal area in a collagenase model of intracerebral hemorrhage (ICH). Apoptosis represents a prominent form of ICH-associated cell death in the perihematomal region and high levels of FAS and FAS ligand transcripts were found in experimental models of hemorrhagic brain. Apoptosis is mainly the regulated cell program for endothelial cell death, but other mechanisms such as necroptosis are also involved.

Cerebral microhemorrhages occur also in COVID-19 patients and could be a consequence of Sars-CoV-2-induced endotheliitis (endothelium inflammation). Several mechanisms have been proposed to explain the increased risk of hemorrhagic stroke (non-ischemic) in COVID-19: (i)The affinity of the SARS-CoV-2 for ACE2 (angiotensin-converting enzyme 2) receptors could allow the virus to directly damage intracranial arteries, causing vessel wall rupture. Also, the downregulation of the renin-angiotensin system may raise blood pressure and put patients already diagnosed with hypertension at higher risk for hemorrhagic stroke. Clinical sequelae in patients with COVID-19 could be explained by the systemic impaired microcirculatory function in different vascular beds. It appears that oxidative stress plays a key role in the pathogenesis of COVID-19 in all direct tissue injury and perpetuates the cytokine storm cycle, and blood clotting mechanism, and exacerbates hypoxia. Excessive ROS may oxidize cellular proteins and lipids, resulting in the prompt destruction of not only virus-infected cells but also healthy cells, which may lead to multiple organ failure including the heart. Induction of apoptosis might have an important role in endothelial cell injury in patients with COVID-19, particularly in pulmonary microvascular endothelial cells and this was correlated with the severity of the disease. Recently, high serum soluble FAS (sFAS) concentrations and low Bcl2 concentrations were associated with mortality of COVID-19 patients.

The hemorrhagic fever has been reported for the cytotoxic effect of other viruses on endothelial cell injury. In dengue hemorrhagic fever patients, apoptosis of microvascular endothelial cells causes plasma leakage, and the soluble FAS ligand (sFasL) acts as a mediator of apoptosis.

Vasculoprotection should be considered to inhibit the hemorrhagic vascular complications leading to death and disability in such pathologies. Especially in cardiac pathologies, vasculoprotection has been neglected in the development of pharmacological treatments, explaining the failure of clinical trials. During thrombotic occlusion of a coronary artery, the ischemic endothelium becomes leaky, leading to extravasation of erythrocytes into the myocardium upon reperfusion. Potential therapeutic strategies for the prevention or attenuation of intramyocardial hemorrhage will include protection of the microvasculature.

Even if it is important to find an effective method to prevent the loss of vascular integrity, no pharmacological treatments targeting myocardial hemorrhages after reperfusion are available. Several preconditioning treatments have been reported to protect vascular integrity and limit hemorrhages but they were administered as pretreatment, so not compatible with AMI patient care nor for the treatment of IR injury. Few drugs evaluated as pharmacological adjuvants of PCI, such as sodium nitroprusside or nicorandil, have shown reducing effects on the no-reflow incidence or increasing cardioprotective properties, respectively, but for both molecules, no protective effects against myocardial hemorrhages have been described.

For the brain, immediate treatment for hemorrhagic stroke is essential for the survival of the patient. Emergency treatment focuses on controlling bleeding and reducing pressure in the brain. Repair of blood vessels is surgically treated. In this case, a small section of the skull is necessary, and medication to reduce blood pressure is prescribed. Regarding pharmacological treatments, a multitude of therapies have been proposed in experimental and clinical studies, such as drugs targeting oxidative stress and components of the blood coagulation cascade, but at present, the clinical benefits for stroke patients are still insufficient. Erythropoietin (EPO) administered for 3 days after ICH induction in an experimental model reduced ICH volume via inhibition of the FAS-dependent pathway, as shown by reduced FAS mRNA levels and inhibition of caspase-8 and caspase-3 activation. However, no description of the EPO effect on endothelial cells has been provided. Previous reports in the literature have shown that preserving endothelial cell viability improved poor outcomes of brain hemorrhagic events such as hemorrhage transformation in animal models of ICH.

However, no antihemorrhagic pharmacological treatment exists to target microvascular injury.

Thus, a need exists for pharmacological treatment to target microvascular injury.

### Description of the invention

The results of Applicant's work lead to the conclusion that a common feature of all hemorrhagic microvascular injury patterns of various origins (viral, oxidative stress, or ischemic) described above, is the involvement of endothelial cell apoptosis.

Furthermore, the Applicant hypothesized that the FAS-dependent pathway is responsible for microhemorrhagic events in the heart.

Following these conclusions and hypothesis, the Applicant has found surprisingly that two interferent peptides may be used as pharmacological treatments to target the extrinsic pathway downstream of the FAS receptor activated in the apoptosis of endothelial cells leading to microhemorrhages, whatever the origin of the injury (viral, oxidative stress, or ischemic).

A proof-of-concept study has been performed with these peptides showing vasculoprotection in two in vitro apoptotic oxidative stress- and hemoglobin-induced models using human endothelial cells as well as in vivo in the mouse model of cardiac injury.

Accordingly, in a first aspect, the present invention provides a conjugate comprising a peptide having the amino acid sequence outlined in SEQ ID NO: 1 (KKSRKEKKQTGSGPLG) or SEQ ID NO: 2 (VGKRKLERVQSGLDL) linked to a cell-penetrating peptide (CPP), for use in the treatment of a condition due to microvascular injury. Peptide having the amino acid sequence outlined in SEQ ID NO: 1 is also named "DAXXp". Peptide having the amino acid sequence outlined in SEQ ID NO: 2 is also named "FADDp".

"peptide" refers herein to the amino acid sequence consisting of SEQ ID NO: 1 or SEQ ID NO: 2, i.e., to the amino acid sequence having no addition, substitution or deletion in sequence SEQ ID NO: 1 or SEQ ID NO: 2. Advantageously, the peptide having the amino acid sequence outlined in SEQ ID NO: 1 ("DAXXp"), which is a fragment of the DAXX protein, interacts with the FAS receptor. Similarly, the peptide having the amino acid sequence outlined in NO: 2 ("FADDp"), which is a fragment of the FADD protein, interacts with the FAS receptor. The peptide can be produced and isolated using any method known in the art, for example by multipin, tea bag, split-couple-mix techniques, or SPOT synthesis.

"Conjugate" refers herein to any cell-penetrating peptide (CPP), which facilitates the uptake of the peptides across cell membranes, such as the plasma membrane of a cell, especially an endothelial cell. CPPs are well-known peptides that can be conjugated to cargo to facilitate transport across the membranes. CPPs are for instance described by Lebleu B. et al. 2008 ([2])). Any CPP can be used to improve the cytoplasmic delivery of fragments or derivatives thereof. Examples of CPPs that can be conjugated with the DAXXp or FADDp, according to the invention may for example be selected from the group consisting of Tat, RXR, Bpep, and Pip2b.

Preferably, the CPP is Tat, which has the amino acid sequence GRKKRRQRRRPPQ (SEQ ID NO: 3). In this case, the conjugate has the amino acid sequence outlined in SEQ ID NO: 4 (GRKKRRQRRRPPQKKSRKEKKQTGSGPLG) also named "Tat-DAXXp", or SEQ ID NO: 5 (GRKKRRQRRRPPQVGKRKLERVQSGLDL), also named "Tat-FADDp". Advantageously, the conjugates, especially Tat-DAXXp conjugate, and Tat-FADDp, are capable of decreasing endothelial cell apoptosis. Advantageously, Tat-DAXXp and Tat-FADDp may have vasculoprotective properties, i.e., may exert an anti-apoptotic effect, especially at the acute phase of the injury, and an increased vascular density compared to the untreated condition. Advantageously, Tat-DAXXp and Tat-FADDp may reduce vascular complications, especially after AMI and reperfusion, hemorrhagic stroke, or viral infection. This treatment advantageously has a positive impact on the risk of death and the risk of developing a chronic disease and disability.

The CPP may be linked to the N-terminal or C-terminal end of the peptide, preferably to the C-terminal. The chemical linkage may be performed via any chemical bond such as for example a disulfide bond, thioether, or thiol-maleimide linkage.

In a particular embodiment, the peptide is linked to the CPP through a linker. Any type of linker can be used by the skilled person, provided that said linker allows chemical linkage to the CPP. A variety of linkers are possible, including amino acid sequences having a C-terminal Cysteine residue that permits the formation of a disulfide, thioether, or thiol-maleimide linkage. Other ways of linking the peptide to the CPP include using a C-terminal aldehyde to form an oxime. Still, other linkers use the Click chemistry.

In an embodiment, the amino acid sequence of the peptide FADDp or DAXXp may comprise substitutions, additions or deletions, which are obvious alternatives for the skilled person, as long as they do not modify the above-mentioned technical effects of the peptide or conjugate. For example, amino acid addition may be another fragment of DAXX protein or of FADD protein.

The terms "DAXX" and "DAXX protein" are used herein interchangeably. They refer to the protein called death-associated protein 6 which, in humans, is encoded by the DAXX gene (RefSeq (mRNA): NM_001141969.1) located at position 21.3 on the short arm (p) of chromosome 6. Generally speaking, DAXX has the amino acid sequence set forth in SEQ ID NO: 6 (shown below). However, the DAXX protein may be an isoform of the DAXX protein of SEQ ID NO: 1 and may therefore have any amino acid sequence that is encoded by the human DAXX gene.
SEQ ID NO: 6:

MATANSIIVL DDDDEDEAAA QPGPSHPLPN AASPGAEAPS
SSEPHGARGS SSSGGKKCYK LENEKLFEEF LELCKMQTAD
HPEVVPFLYN RQQRAHSLFL ASAEFCNILS RVLSRARSRP
AKLYVYINEL CTVLKAHSAK KKLNLAPAAT TSNEPSGNNP
PTHLSLDPTN AENTASQSPR TRGSRRQIQR LEQLLALYVA
EIRRLQEKEL DLSELDDPDS AYLQEARLKR KLIRLFGRLC
ELKDCSSLTG RVIEQRIPYR GTRYPEVNRR IERLINKPGP
DTFPDYGDVL RAVEKAAARH SLGLPRQQLQ LMAQDAFRDV
GIRLQERRHL DLIYNFGCHL TDDYRPGVDP ALSDPVLARR
LRENRSLAMS RLDEVISKYA MLQDKSEEGE RKKRRARLQG
TSSHSADTPE ASLDSGEGPS GMASQGCPSA SRAETDDEDD
EESDEEEEEE EEEEEEEATD SEEEEDLEQM QEGQEDDEEE
DEEEEAAAGK DGDKSPMSSL QISNEKNLEP GKQISRSSGE
QQNKGRIVSP SLLSEEPLAP SSIDAESNGE QPEELTLEEE
SPVSQLFELE IEALPLDTPS SVETDISSSR KQSEEPFTTV
LENGAGMVSS TSFNGGVSPH NWGDSGPPCK KSRKEKKQTG

SGPLGNSYVE RQRSVHEKNG KKICTLPSPP SPLASLAPVA
DSSTRVDSPS HGLVTSSLCI PSPARLSQTP HSQPPRPGTC
KTSVATQCDP EEIIVLSDSD

The terms "FADD" and "FADD protein" are used herein interchangeably. They refer to the protein called Fas-Associated protein with Death Domain which, in humans, is encoded by the FADD gene (RefSeq (mRNA): NM_003824.3) located at position 13.3 on the long arm (q) of chromosome 11. Generally speaking, FADD has the amino acid sequence set forth in SEQ ID NO: 7 (shown below). However, the FADD protein may be an isoform of the FADD protein of SEQ ID NO: 7 and may therefore have any amino acid sequence that is encoded by the human FADD gene.
SEQ ID NO: 7:

MDPFLVLLHS VSSSLSSSEL TELKFLCLGR VGKRKLERVQ
SGLDLFSMLL EQNDLEPGHT ELLRELLASL RRHDLLRRVD
DFEAGAAAGA APGEEDLCAA FNVICDNVGK DWRRLARQLK
VSDTKIDSIE DRYPRNLTER VRESLRIWKN TEKENATVAH
LVGALRSCQM NLVADLVQEV QQARDLQNRS GAMSPMSWNS
DASTSEAS

« Microvascular injury » refers herein to endothelial cell damages causing disease of the microvessels, small blood vessels in the microcirculation. In this context, apoptosis of endothelial cells is an important mechanism of vascular injury, resulting in vascular leak, inflammation, and coagulation.

In an embodiment, the microvascular injury may be a microhemorrhage. In this case, a microhemorrhage may be a condition chosen among Intra Cerebral Hemorrhage (ICH), hypertension hemorrhage, sub-arachnoid hemorrhage (SARAH), hemorrhage due to retinopathy of prematurity (ROP), hemorrhage due to proliferative diabetic retinopathy (PDR), dengue hemorrhage fiver, intramyocardial bleeding after Acute myocardial infarction (AMI), intracranial hemorrhage after stroke, and hemorrhage after COVID-19 infection.

Alternatively, the condition may be a no-reflow phenomenon.

In an embodiment, the conjugate may be in a pharmaceutical composition comprising an effective amount of at least one conjugate as defined above, and at least one pharmaceutically acceptable carrier or excipient. Thus, another object of the invention relates to a pharmaceutical composition comprising an effective amount of at least one conjugate as defined above, and at least one pharmaceutically acceptable carrier or excipient, for use in the treatment of a condition due to microvascular injury.

The term "pharmaceutically acceptable carrier or excipient" refers to a carrier medium that does not interfere with the effectiveness of the biological activity of the active ingredient(s) and which is not significantly toxic to the host at the concentration at which it is administered. The term includes solvents, dispersion, media, coatings, antibacterial and antifungal agents, isotonic agents, and adsorption delaying agents, and the like. The use of such media and agents for pharmaceutically active substances is well known in the art.

Also described is a method for treating a disease or condition associated with endothelial apoptosis in a subject, comprising a step of administering to said subject an effective amount of at least one conjugate as defined above, or a pharmaceutical composition as described above, wherein said disease or condition associated with endothelial apoptosis is a microvascular injury as define above.

As used herein, "subject" refers to a human or animal that may benefit from the administration of at least one conjugate as defined above. By a "therapeutically effective amount" according to the invention, it is meant a sufficient amount to treat the disease, at a reasonable benefit/risk ratio applicable to any medical treatment.

This invention is further illustrated by the following examples with regard to the annexed drawings that should not be construed as limiting.

### Brief description of the figures

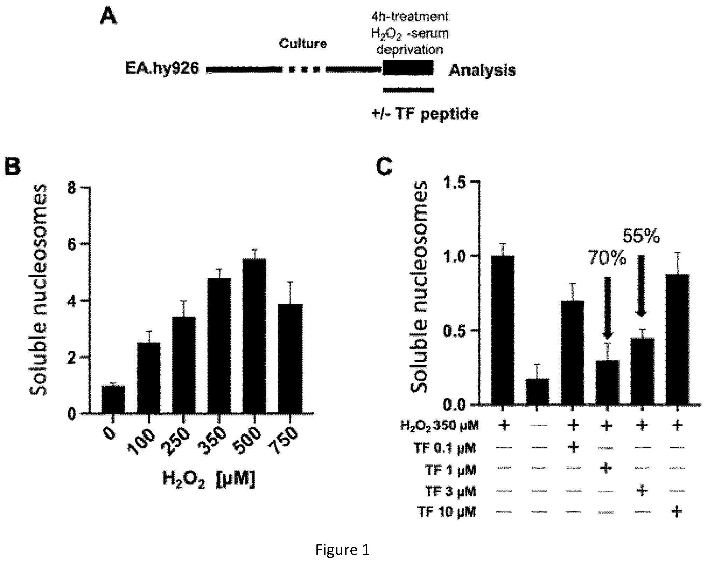

- Figure 1 represents Tat-FADDp inhibiting oxidative stress-induced apoptosis in endothelial cells. (A) Experimental and treatment protocol of acute stress-induced apoptosis. Tat-FADDp (TF) was added to endothelial EA.hy926 cells in the medium containing H₂O₂ without serum. (B) Hydrogen peroxide (H₂O₂) dose-response to identify the optimal H₂O₂ treatment for reversible oxidative stress-induced apoptosis in endothelial EA.hy926 cells measured as specific DNA fragmentation (soluble nucleosomes normalized to 0 µM H₂O₂). Data represent the mean ± SD of values obtained for n= 2 independent cultures. (C) Dose-dependent anti-apoptotic effect of TF (0.1, 1, 3 and 10 µM) in endothelial EA.hy926 cells with H₂O₂ stress-induced apoptosis (350 µM H₂O₂ /4 h with deprivation in serum) measured as specific DNA fragmentation (soluble nucleosomes normalized to 350 µM H₂O₂). Data represent the mean ± SD of values obtained for n= 2 independent cultures.

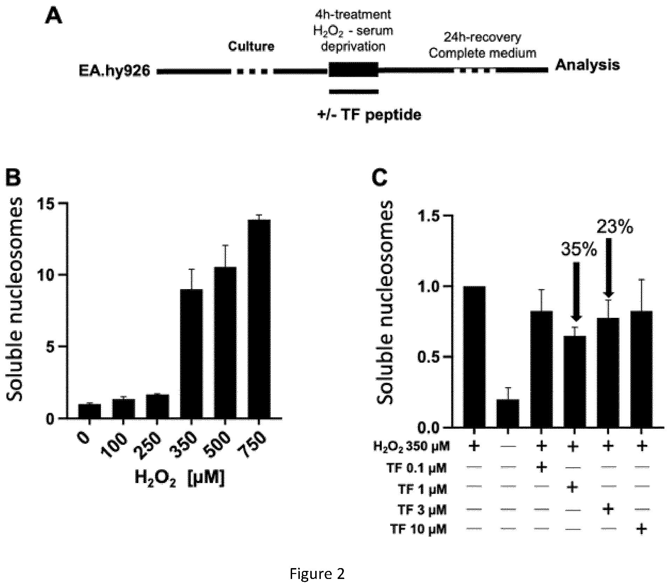

- Figure 2 represents Tat-FADDp inhibiting oxidative stress-induced apoptosis in endothelial cells with 24 h recovery. (A) Experimental and treatment protocol of stress-induced apoptosis with 24 h of recovery with complete medium. Tat-FADDp (TF) was applied during the induction of the apoptotic stress in endothelial EA.hy926 cells. (B) Hydrogen peroxide (H₂O₂) dose-response to identify the optimal H₂O₂ dose-inducing reversible oxidative stress-induced apoptosis with 24 h recovery in endothelial EA.hy926 cells measured as specific DNA fragmentation (soluble nucleosomes normalized to 0 µM H₂O₂). Data represent the mean ± SD with n= 2 independent cultures. (C) Dose-dependent anti-apoptotic effect of TF (0.1, 1, 3 and 10 µM) in endothelial EA.hy926 cells with stress-induced apoptosis (350 µM H₂O₂/4h with serum deprivation) followed by a 24 h-period of recovery with complete medium assessed as specific DNA fragmentation (soluble nucleosomes normalized to 350 µM H₂O₂). Data represent the mean ± SD with n= 2 independent cultures.

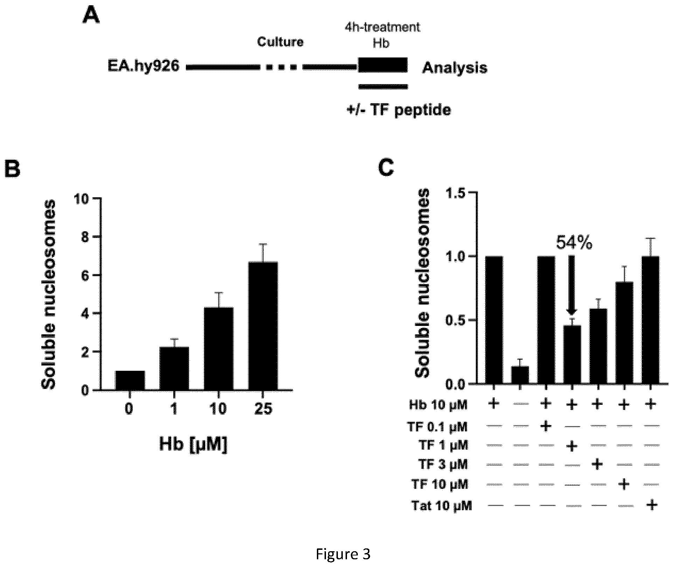

- Figure 3 represents Tat-FADDp inhibiting Hemoglobin-induced apoptosis in endothelial cells. (A) Experimental and treatment protocol of stress-induced apoptosis upon Hemoglobin (Hb) exposure. Tat-FADDp (TF) was applied during the induction of the apoptotic stress. (B) Hb dose-response to identify the optimal Hb dose inducing reversible stress-induced apoptosis in endothelial EA.hy926 cells measured as specific DNA fragmentation (soluble nucleosomes normalized to 0 µM Hb). Data represent the mean ± SD of n=2 independent cultures. (C) Dose-dependent anti-apoptotic effect of TF (1, 3, 10, and 30 µM) in endothelial EA.hy926 cells with stress-induced apoptosis (10 µM Hb /4h with serum deprivation) assessed as specific DNA fragmentation (soluble nucleosomes normalized to 10 µM Hb). Tat (T, 10 µM) used as a control peptide has no protective effect on the Hb-induced apoptosis.

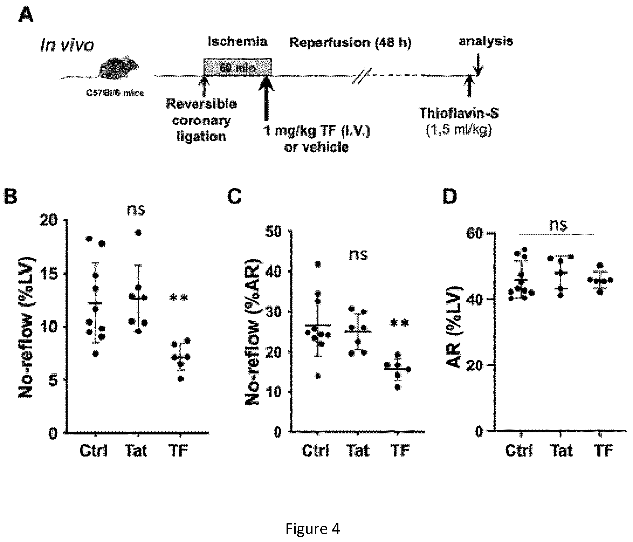

- Figure 4 represents Tat-FADDp decreasing the no-reflow phenomenon after myocardial reperfusion. (A) Experimental and treatment *in vivo* protocol to evaluate the no-reflow phenomenon. Mice were subjected to 60 minutes of ischemia (I) after the left coronary artery ligation, myocardial reperfusion (R) was achieved by releasing the knot and mice were awaked after suturing the chest. Tat (1 mg/kg) or Tat-FADDp (TF; 1 mg/kg) was injected intravenously 5 minutes before the onset of reperfusion. 48 h later, anesthesia was induced again, Thioflavin-S was injected, and the coronary ligation tightened definitively before the injection of the phthalocyanine blue dye. The heart was removed for histological analysis. The control group received intravenous saline injection (vehicle). (B, C, D) Scatter dot plots and mean ± SD for no-reflow calculated as a percentage of (B) the left ventricle (LV) or (C) of the area at risk (%AR). In panel (D) the AR calculated as a percentage of LV for each heart subjected to the protocol is presented. Statistical analysis was performed using the Kruskal-Wallis non-parametric test for multiple comparisons versus control (Ctrl). For no-reflow (%LV), ** was noted for p=0.0092 and ns for p>0.999; for no-reflow (%AR), ** was noted for p=0.0084 and ns for p>0.999; for AR (%LV), ns was noted for p=0.6682.

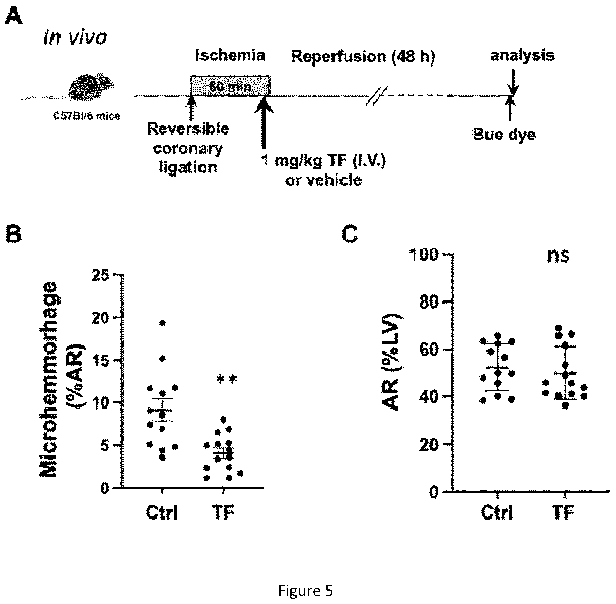

- Figure 5 represents Tat-FADDp decreasing microhemorrhages after myocardial reperfusion. (A) Experimental and treatment protocol to identify hemorrhagic events in mice subjected to 60 min ischemia and treated with Tat-FADDp (TF; 1 mg/kg) intravenously 5 min before reperfusion followed by 48 h-myocardial reperfusion. The control group received intravenous saline injection (vehicle). (B, C) Scatter dot plots and mean ± SD for microhemorrhage volume calculated as a percentage of the ischemic area at risk (%AR) (B) and for AR calculated as a percentage of LV (C). Statistical analysis was performed using an unpaired T-test (Normality test passed) for microhemorrhage data or Mann-Whitney (AR data). p values were noted ** for p=0.0011 and ns for p=0.6848 versus Ctrl.

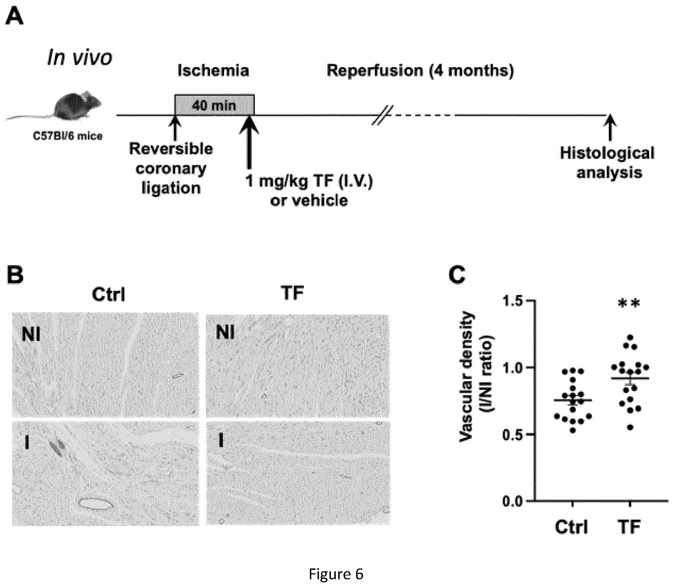

- Figure 6 represents Tat-FADDp increasing the vascular density in infarcted hearts in the long term. (A) Experimental and treatment protocol to identify vasculoprotection in mice after 50 minutes of ischemia and 6-month-myocardial reperfusion. Tat-FADDp (TF, 1 mg/kg) was injected as a single intravenous bolus 5 min before reperfusion. The control group received intravenous saline injection (vehicle). At the end of the protocol, hearts were harvested and a histological analysis was performed. (B)Representative pictures of microscopic observations for CD31-stained sections from Ctrl and TF treated hearts both in ischemic (I) and non-ischemic (NI) areas. (C) Vascular density (I/NI ratio) was increased in TF-treated *versus* Ctrl LV samples. Data represent the scatter dot plots and mean ± SEM. Statistical analysis was performed using an unpaired T-test (Normality test passed). p-value was noted ** for p=0.0079 *versus* Ctrl.

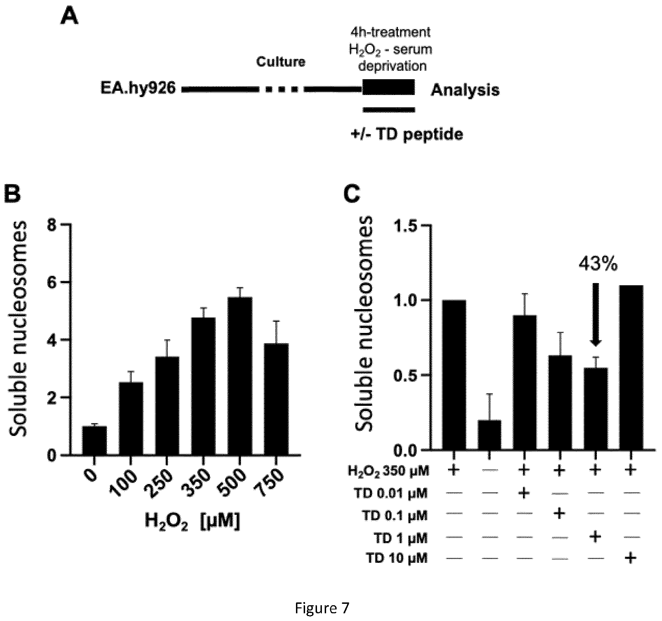

- Figure 7 represents Tat-DAXXp inhibiting oxidative stress-induced apoptosis in endothelial cells. (A) Experimental and treatment protocol of acute stress-induced apoptosis. Tat-DAXXp (TD) was incubated in the medium containing H₂O₂ without serum. (B) H₂O₂ dose-response to identify the optimal H₂O₂ treatment for reversible stress-induced apoptosis in endothelial EA.hy926 cells measured as specific DNA fragmentation (soluble nucleosomes normalized to 0 µM H₂O₂). Data represent the mean ± SD with n= 2 independent cultures. (C) Dose-dependent anti-apoptotic effect of TD (0.01, 0.1, 1, and 10 µM) in endothelial EA.hy926 cells with H₂O₂ stress-induced apoptosis (350 µM H₂O₂ / 4 h with deprivation in serum) assessed by specific DNA fragmentation quantification (soluble nucleosomes normalized to 350 µM H₂O₂). Data represent the mean ± SD with n= 3 independent cultures.

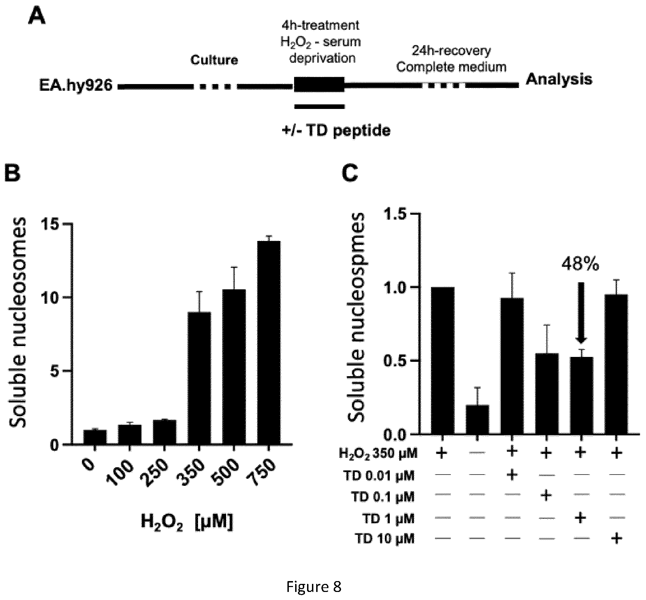

- Figure 8 represents Tat-DAXXp inhibiting oxidative stress-induced apoptosis in endothelial cells with recovery. (A) Experimental and treatment protocol of stress-induced apoptosis with 24 h recovery in complete medium. (B) H₂O₂ dose-response to identify the optimal H₂O₂ treatment for a reversible stress-induced apoptosis with 24 h recovery in endothelial EA.hy926 cells measured as specific DNA fragmentation (soluble nucleosomes normalized to 0 µM H₂O₂). Data represent the mean ± SD with n= 2 independent cultures. (C) Dose-dependent anti-apoptotic effect of TD (0.01, 0.1, 1 and 10 µM) in endothelial EA.hy926 cells with H₂O₂ stress-induced apoptosis (350 µM H₂O₂ /4 h with deprivation in serum) measured as specific DNA fragmentation (soluble nucleosomes normalized to 350 µM H₂O₂). Data represent the mean ± SD with n= 2 independent cultures.

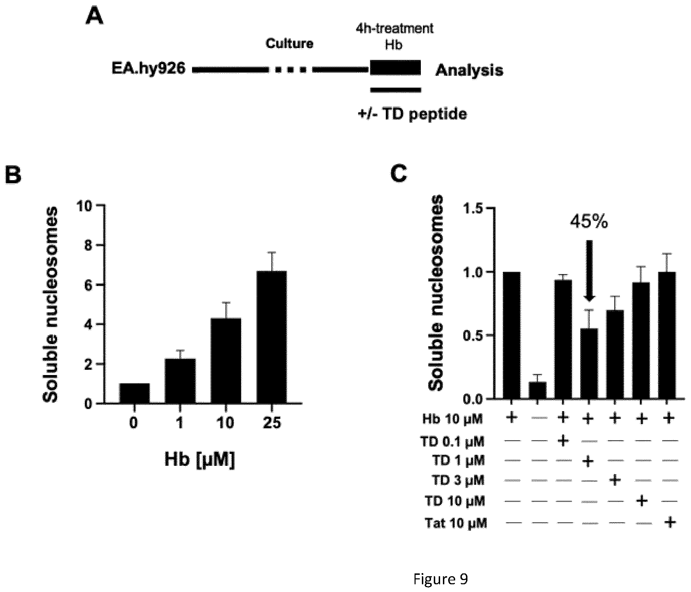

- Figure 9 represents Tat-DAXXp inhibiting Hb-induced apoptosis in endothelial cells. (A) Experimental and treatment protocol of stress-induced apoptosis upon Hb exposure. Tat-DAXXp (TD) was applied during the induction of the apoptotic stress. (B) Hb dose-response to identify the optimal Hb dose inducing reversible stress-induced apoptosis in endothelial EA.hy926 cells measured as specific DNA fragmentation (soluble nucleosomes normalized to 0 µM Hb) in the absence of TD. Data represent the mean ± SD of n=2 independent cultures. (C) Dose-dependent anti-apoptotic effect of TD (0.1, 1, 3 and 10 µM) in endothelial EA.hy926 cells with stress-induced apoptosis (10 µM Hb /4h with serum deprivation) assessed as specific DNA fragmentation (soluble nucleosomes normalized to 10 µM Hb). Data represent the mean ± SD of n=3 independent cultures.

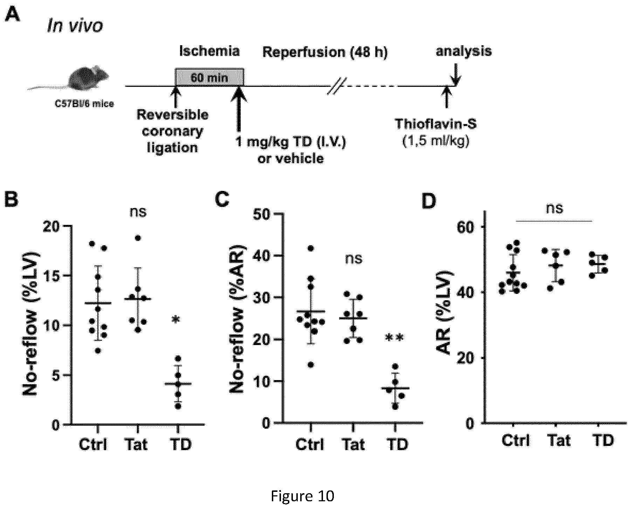

- Figure 10 represents Tat-DAXXp decreasing the no-reflow phenomenon after myocardial reperfusion. (A) Experimental and treatment protocol to identify no-reflow in mice subjected to 60 min ischemia and treated with Tat (1 mg/kg) or Tat-DAXXp (TD, 1 mg/kg) intravenously 5 min before reperfusion followed by 48 h-myocardial reperfusion. The control group received intravenous saline injection (vehicle). (B, C, D) Scatter dot plots and mean ± SD are presented for the no-reflow calculated as a percentage of (B) the left ventricle (%LV) or (C) of the area at risk (%AR). In panel (D) the AR calculated as a percentage of LV for each heart subjected to the protocol is presented. Statistical analysis was performed using the Kruskal-Wallis non-parametric test for multiple comparisons versus control (Ctrl). For no-reflow (%LV), * was noted for p=0.0104 and ns for p>0.999; for no-reflow (%AR), ** was noted for p=0.0045 and ns for p>0.999; for AR (%LV), ns was noted for p=0.6092.

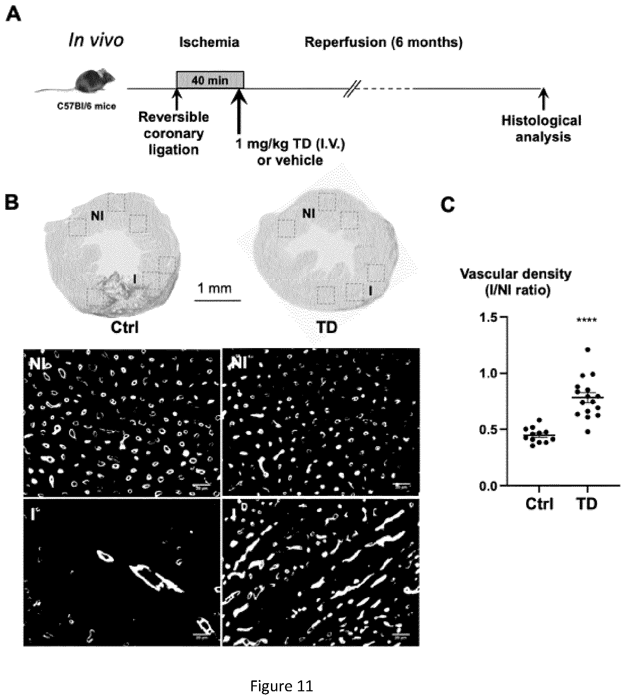

- Figure 11 represents Tat-DAXXp increasing the vascular density in infarcted hearts in the long term. (A) Experimental and treatment protocol to identify vasculoprotection in mice after 40 minutes of I and 6 month-myocardial reperfusion. Tat-DAXXp (TD, 1 mg/kg) was injected as a single intravenous bolus 5 min before reperfusion. The control group received intravenous saline injection (vehicle). At the end of the protocol, hearts were harvested and a histological analysis was performed. The presence of microvessels was detected in LV tissue by the mean of co-immunostaining with Isolectin B4 and DAPI. (B): Representative pictures of microscopic observations (Scale bar: 1 mm) for SHAM, Ctrl, and TD-treated LV after picrosirius staining. Sections (dotted squares) were highlighted with counting areas selected for the determination of isolectin B4 positive vessels in both the non-ischemic and the ischemic parts of the LV. Representative enlarged immunostaining images (Original magnification: x40 oil immersion) are presented showing vessels (isolectin B4) in the non-ischemic (NI, upper panel) and the ischemic (I; lower panel) regions; Scale bar: 20 µM. (C): Isolectin B4-positive vessels were determined by randomly counting in n=3 zones of the border zone and n=3 zones of the remote area from Ctrl (n=16) and TD (n=12) LVs. Data were compared using T-test. **** was noted for p<0.0001 versus Ctrl.

### EXAMPLES

### Example 1: Tat-FADDp inhibits oxidative stress-induced apoptosis in endothelial cells

The protocol (See Figure 1A) consisted of exposing EA.hy926 endothelial cells to various concentrations of H₂O₂ under serum deprivation for 4 h to induce oxidative stress. The anti-apoptotic peptide Tat-FADDp (TF) was incubated during the apoptotic stimuli at various concentrations (0.1, 1, 3, and 10 µM) and specific DNA fragmentation (detected as soluble nucleosomes) was quantified at the end of the protocol.

First, as illustrated in Figure 1B, we established the dose-response and identified 350 µM as the optimal dose of H₂O₂ allowing a strong but not maximal induction of apoptosis (= reversible apoptosis).

Co-incubating the Tat-FADDp peptide (at various concentrations ranging from 0.1 to 10 µM) with H₂O₂ allowed the specific reduction of DNA fragmentation up to 70% (see Figure 1C); 1 µM Tat-FADDp was the optimal dose providing the maximal inhibition of apoptosis.

Then, specific DNA fragmentation (detected as soluble nucleosomes) was quantified in EA.hy926 cells treated with H₂O₂ during 4 h under serum deprivation followed by a recovery in the complete medium during 24 h after the stress induction (See protocol in Figure 2A). Again, the dose-response for H₂O₂ treatment was investigated to select the dose of 350 µM among others for its efficiency (Figure 2B). Then Tat-FADDp peptide (at various concentrations ranging from 0.1 to 10 µM) was co-incubated during 4 h H₂O₂ exposure followed by a recovery period of 24 h. The maximal anti-apoptotic effect (-35% versus the H₂O₂ condition) was obtained at an optimal dose of 1 µM Tat-FADDp (Figure 2C).

### Example 2: Tat-FADDp inhibits apoptosis in an in vitro model of hemorrhage

We used an in vitro model of cytotoxicity induced by human hemoglobin (Hb) considered as an in vitro model of cell death induction during hemorrhage (see the protocol of Figure 3A) (Imai et al. 2019 ([3])).

First, we established the dose-response for Hb (1, 10, 25 µM) applied during 4 h (Figure 3B) to confirm the previously reported apoptotic rate described for endothelial cells after Hb treatment (Imai et al. 2019 ([3])). Indeed, Figure 3B shows that Hb treatment significantly induced cell death in EA.hy926 cells in a concentration-dependent manner. We selected the dose of 10 µM of Hb to evaluate the anti-apoptotic effect of Tat-FADDp (TF) at various concentrations (0.1, 1, 3, and 10 µM). Tat-FADDp 1 µM (optimal dose) was able to reduce Hb-induced apoptosis by 54% compared to the Hb-treated condition, by contrast to Tat (10 µM), which did not affect apoptosis (Figure 3C).

### Example 3: Vasculoprotective effect of Tat-FADDp in vivo decreased no-reflow phenomenon and hemorrhages

Around 50% of reperfused MI patients develop MVO and 40% of intramyocardial hemorrhage (IMH), which are associated with larger infarct size, adverse left ventricle (LV) remodeling, and worse clinical outcomes. Indeed, cardiac vessels are not spared in IR injury, and in the most severe form, capillary destruction with hemorrhage occurs. Therefore, vascular integrity and hemorrhages were investigated on LV tissues subjected to IR in vivo and treated by Tat-FADDp to evaluate its vasculoprotective effects. To do this, effects of Tat-FADDp (1 mg/kg, IV 5 min before R) were analyzed in mice subjected to 60 min of ischemia followed by 48 h of reperfusion, a period necessary to get vascular injury since it has been reported in the mouse heart that death of vascular cells (vascular rhexis) in non-sustained ischemia occurs after 45 h (see protocol presented in Figure 4A). To quantitatively measure MVO, Thioflavin-S, a dye that stains perfused endothelium, was injected before harvesting the heart at the end of the protocol.

The no-reflow expressed as a percentage of the LV (Figure 4B) or as a percentage of the area at risk (Figure 4C) was significantly decreased in the Tat-FADDp-treated (-41%) versus non-treated (Ctrl) or Tat-treated IR mice. As an additional control, the area at risk as a percentage of LV revealed that cardiac IR injury was similar among all groups (Figure 4D).

To evaluate hemorrhagic events, mice were subjected to 60 min of ischemia followed by 48 h of reperfusion (Figure 5A). The histological analysis revealed intra-myocardial hemorrhage in the central core of the infarct. The hemorrhage volume was measured and expressed as a percentage of the area at risk (Figure 5B). Tat-FADDp (1 mg/kg, IV 5 min before R) significantly decreased the hemorrhage extent (-55%) versus the control condition in non-treated IR hearts (Ctrl). As an additional control, the area at risk (% of LV) was similar among groups revealing that cardiac IR injury was the same in the two conditions (Figure 5C).

### Example 4: Tat-FADDp restores vascular density in the myocardium 4 months after myocardial injury

In this study, mice subjected to a 40 min ischemia (reversible coronary ligation) were allowed to recover 4 months after reperfusion. After this follow-up, hearts were removed and histological analysis was performed to count the number of microvessels after CD31 staining (a marker specific of endothelial cells; Figure 6A) in the border zone and the remote area within the myocardial thickness. Indeed, the vascular density in the border zone of infarct was increased in the Tat-FADDp treated group versus control (+22%) showing that Tat-FADDp (1 mg/kg, IV 5 min before R) provides long-term vasculoprotection (Figures 6B, C). For the first time, we were able to demonstrate that Tat-FAXXp allowed to restore the ventricular vascularization network in the myocardium after myocardial infarction and reperfusion.

### Example 5 : Tat-DAXXp inhibits oxidative stress-induced apoptosis in endothelial cells

The protocol (See Figure 7A) consisted in exposing EA.hy926 endothelial cells to various concentrations of H₂O₂ under serum deprivation for 4 h to induce oxidative stress. Tat-DAXXp (TD) was incubated during the apoptotic stimuli at various concentrations (0.01, 0.1, 1, and 10 µM) and specific DNA fragmentation was quantified at the end of the protocol.

First, as illustrated in Figure 7B, we established the dose-response and identified 350 µM as the optimal dose of H₂O₂ allowing a strong but not maximal induction of apoptosis (= reversible apoptosis).

Co-incubating the Tat-DAXXp peptide (at various concentrations ranging from 0.01 to 10 µM) with H₂O₂ allowed to reduce specific DNA fragmentation by 43% (see Figure 7C); 1 µM Tat-DAXXp was the optimal dose providing the maximal inhibition of apoptosis.

Then, specific DNA fragmentation was quantified in EA.hy926 cells treated with various concentrations of H₂O₂ during 4 h under serum deprivation followed by a recovery in complete medium during 24 h after the oxidative stress (See protocol in Figure 8A). Again, the dose response for H₂O₂ treatment was investigated (Figure 7B) allowing us to choose 350 µM of H₂O₂ as the working concentration to induce reversible apoptosis in endothelial cells. Then, Tat-DAXXp (TD) was applied at various concentrations ranging from 0.01 to 10 µM during the 4 h of the apoptotic stimulus. The maximal anti-apoptotic effect (-48% versus the H₂O₂ condition) was obtained at 1 µM Tat-DAXXp (Figure 8C).

### Example 6: Tat-DAXXp inhibits apoptosis in an in vitro model of hemorrhage

We used an in vitro model of cytotoxicity induced by human hemoglobin (Hb) considered as an in vitro model of cell death induction during hemorrhage (see the protocol of Figure 9A) (Imai et al. 2019 ([3])).

First, we established the dose-response for Hb (1, 10, 25 µM) applied during 4 h and we assessed the apoptotic rate of endothelial cells after Hb treatment. Figure 9B shows that Hb treatment significantly induced cell death in EA.hy926 cells in a concentration-dependent manner as reported previously (Imai et al. 2019 ([3])). We selected the dose of 10 µM of Hb to evaluate the anti-apoptotic effect of TD. Various concentrations of TD were tested (0.1, 1, 3, and 10 µM). Tat-DAXXp 1 µM (optimal dose) was able to reduce Hb-induced apoptosis by 45% compared to the Hb-treated condition, by contrast to Tat (10 µM), which did not affect apoptosis (Figure 9C).

### Example 7 : Tat-DAXXp decreases the no-reflow phenomenon after myocardial reperfusion

No-reflow was evaluated on LV tissues subjected to IR in vivo and treated by Tat-DAXXp to evaluate its vasculoprotective effect. To do this, effects of Tat-DAXXp (1 mg/kg, IV 5 min before R) were analyzed in an IR-mouse model subjected to 50 min of ischemia followed by 48 h of reperfusion, a period necessary to get vascular injury since it has been reported in the mouse heart that death of vascular cells (vascular rhexis) in non-sustained ischemia occurs after 45 h (see protocol presented in Figure 10A). To quantitatively measure MVO, Thioflavin-S, a dye that stains perfused endothelium, was injected before harvesting the heart at the end of the protocol.

Figure 10 shows that the no-reflow, expressed as a percentage of the LV (B) or as a percentage of the area at risk (C) was significantly decreased versus the control condition in hearts subjected to the same injury as illustrated by Figure 10D presenting the similar values of AR (%LV) among all groups. Tat alone did not provide any protective effects.

### Example 8: Tat-DAXXp restores the vascular density in reperfused infarcted hearts on the long term

Following the failure of clinical trials in cardioprotection, recommendations from clinicians have been made to consider the coronary circulation as a target of cardioprotection. To evaluate the vasculoprotective effect of Tat-DAXXp in a clinical context with long periods of reperfusion for AMI patients, we decided to quantify the vascular density in IR hearts treated by one bolus of Tat-DAXXp (1 mg/kg) at the time of reperfusion after a 6 month-period of reperfusion. To do this, we performed an analysis by immunochemistry using isolectin B4 as specific positive vessels were counted both in the border zone of the infarct and the non-ischemic remote areas of the LVs. As illustrated in Figure 11, the Tat-DAXXp treatment was able to increase the vascular density by 75% compared to that observed in the non-treated Ctrl LV (p**** <0.0001 for TD, n=16 versus Ctrl, n=12; Figures 11 B, C). For the first time, we were able to demonstrate that Tat-DAXXp allowed to restore the ventricular vascularization in the post-infarction period.

### Material and methods in relation to examples 1 to 8

### Endothelial cell culture

Human umbilical vein endothelial cells EA.hy926 (ATCC) were cultured in Dulbecco's modified Eagle's medium (DMEM, (Gibco), supplemented with 10% fetal bovine serum (FBS, Gibco), 100 Units/mL penicillin, and 100 µg/mL streptomycin (Gibco). To induce apoptosis, EA.hy926, cells were cultured in a minimal medium (without FBS) containing H₂O₂ (100, 250, 350, 500, and 750 µM, Sigma Aldrich) or with hemoglobin (Hb, 1 µM, 10 µM, and 25 µM, Sigma Aldrich) for 4 h.

### DNA fragmentation assay

Specific DNA fragmentation as a hallmark of apoptosis was quantified in vitro on cell lysates with an enzyme-linked immunosorbent assay kit (Cell Death ELISA; Roche Diagnostics) designed to measure histone-complexed DNA fragments (mono- and oligonucleosomes) released in the cytosol of cells induced for apoptosis. Data were normalized as indicated in the figure legends.

### Animals

All experiments were carried out on C57BL/6J mice (Charles River Laboratory) following the European Communities Council Directive of November 1986 and conformed to the "Guide for the Care and Use of Laboratory Animals" published by the US National Institutes of Health (NIH publication 8th Edition, 2011). The study protocols were approved by the "Comité d'éthique pour l'expérimentation animale Languedoc-Roussillon" (CEEA-LR) with the authorization number CEEA-LR-12107 for experiments to be realized on the site of experimentation that obtained authorization number A 34-172-41.

### Surgery

Investigations conform to the Directive 2010/63/EU of the European Parliament. C57Bl6J male mice were subjected to a surgical model of acute myocardial ischemia and reperfusion as previously described (Roubille et al. 2011 ([4]); Vincent et al. 2017 ([5])). Mice (22-30 g, Charles River, France) were anesthetized by an anesthetic preparation (intramuscular injection) composed of xylazine (10 mg/kg; Rompun 2%; Bayer), ketamine (50 mg/kg; Imalgene 500; Merial) and chlorpromazine (1.25 mg/kg; Largactil 5 mg/mL; Sanofi-Aventis). Mice were placed under mechanical ventilation through tracheal intubation on a rodent respirator (Harvard Apparatus, tidal volume/body mass: 7.2 µL/g; respiratory rate: 200 breaths per min). A thermo-regulated table (connected to a rectal probe) allowed maintaining the body temperature between 36.8 and 37°C during the surgery. Ketamine (50 mg/kg; Imalgene 500, Merial) and xylazine (10 mg/kg, Rompun 2%; Bayer) were injected to fully induce anesthesia before opening the chest by a left lateral thoracotomy and placing a reversible coronary artery snare occluder around the left coronary artery. During the surgical protocol, anesthesia and pain analgesia were controlled by pinching the toes of the posterior legs. All mice experienced an ischemic injury of 40 min. The coronary reperfusion was obtained by loosening the silk knot. Then, the chest was sutured after administration of lidocaine (1.5 mg/kg; Xylocaine 10 mg/mL; AstraZeneca) subcutaneously. Postoperative awakening was achieved with oxygen supply and controlled humidity in an emergency care unit maintained at 28°C where mice were allowed to recover for 24 h. Then they were transferred to a ventilated cabinet where they were maintained for 24 h for the no-reflow evaluation and for 6 months for the long-term evaluation of vasculoprotection.

Various protocols were applied:
- SHAM: sham operation for surgery placebo was performed without coronary artery ligation (surgical placebo).
- Ctrl (non-treated IR mice): 40/60 min ischemia - 48 h/4 months/6 months reperfusion with an I.V. injection of physiological serum 5 min before the onset of reperfusion (pharmacological placebo).
- TD/TF (TD/TF-treated IR mice): 40/60 min ischemia - 48 h/4 months/6 months with an I.V. injection of TD/TF peptide (1 mg/kg) 5 min before the onset of reperfusion (pharmacological treatment).

Mice subjected to the IR protocol were randomly allocated to the two groups of treatment Ctrl or TD (blind to the experimenter).

### Pharmacological treatments

Tat-DAXXp (TD), Tat-FADDp (TF), and Tat (from Intavis AG or LifeTein) were solubilized in physiological serum (NaCl 0.9%; Lavoisier). For the cell experiments, peptide solutions of 1 mM were prepared and diluted to indicated concentration in the medium.

For the in vivo experiments, peptide solutions of 10 mg/mL were prepared. Pharmacological treatments with 15 µL of the peptide solution (final concentration of 1 mg/kg) or physiological serum (NaCl 0.9%; Lavoisier) were given blindly to the surgery experimenter, who administered them intravenously in the tail vein of the anesthetized mice 5 min before reperfusion.

### Histological analysis of No-reflow, hemorrhages, and infarct size

After 48 h of reperfusion, Thioflavin-S (4%; 1.5 ml/kg (Sigma Aldrich) (Galaup et al. 2012 ([6])) fluorescent vital stain for endothelium (which shows bright blue-green fluorescence under 420-nm UV light), was injected in mice anesthetized with xylazine (10 mg/kg; Rompun 2%; Bayer) and ketamine (50 mg/kg; Imalgène 500; Merial). Then, the coronary ligation threads left in place during the infarct surgery and the following 48 h-reperfusion period were definitely tightened and the phthalocyanine blue dye was injected. The heart was removed, and rinsed with cold PBS. The left ventricle (LV) was dissected out and cut into 1 mm thick slices. Each side of the slices was photographed under visible and ultraviolet light to assess hemorrhagic red and no reflow areas, respectively. LV slices were incubated in 2,3,5-triphenyltetrazolium chloride (15 min, 37°C; Sigma Aldrich) to stain the viable part of the myocardial tissue in brick red. Each side of the slices was photographed. No-reflow and infarct size were measured using Image J software and expressed as a percentage of the ischemic area at risk (Galaup et al. 2012 ([6])).

### Immunochemistry analysis

At the end of the surgery, the heart will be harvested, the LV was fixated in paraformaldehyde and transferred to the Biocampus RHEM histology platform (https://www.rhem.cnrs.fr/) for paraffin embedding and 4 µm slicing for immunochemistry experiments. The integrity of microvasculature was evaluated using either isolectin B4 FITC conjugate (Sigma Aldrich) or anti-CD31 (Abcam) for endothelial cell labeling on LV slices from treated versus non-treated mice. The stained slides will be scanned on a whole slide scanner (Nanozoomer 2.0-HT, Hamamatsu) to acquire whole slide imaging at ×20 magnification.

### REFERENCE LIST

1. Roubille, F., S. Combes, J. Leal-Sanchez, C. Barrere, F. Cransac, C. Sportouch-Dukhan, G. Gahide, I. Serre, E. Kupfer, S. Richard, A. O. Hueber, J. Nargeot, C. Piot and S. Barrere-Lemaire (2007). "Myocardial expression of a dominant-negative form of Daxx decreases infarct size and attenuates apoptosis in an in vivo mouse model of ischemia/reperfusion injury." Circulation 116(23): 2709-2717.
2. Lebleu B. et al., Advanced Drug Delivery Reviews 60 (2008) 517-529 and by Said Hassane F. et al, Cell. Mol. Life Sci. (2010) 67:715-726.
3. Imai, T., S. Iwata, T. Hirayama, H. Nagasawa, S. Nakamura, M. Shimazawa and H. Hara (2019). "Intracellular Fe(2+) accumulation in endothelial cells and pericytes induces blood-brain barrier dysfunction in secondary brain injury after brain hemorrhage." Sci Rep 9(1): 6228.
4. Roubille, F., A. Franck-Miclo, A. Covinhes, C. Lafont, F. Cransac, S. Combes, A. Vincent, P. Fontanaud, C. Sportouch-Dukhan, C. Redt-Clouet, J. Nargeot, C. Piot and S. Barrere-Lemaire (2011). "Delayed postconditioning in the mouse heart in vivo." Circulation 124(12): 1330-1336.
5. Vincent, A., A. Covinhes, C. Barrere, L. Gallot, S. Thoumala, C. Piot, C. Heurteaux, M. Lazdunski, J. Nargeot and S. Barrere-Lemaire (2017). "Acute and long-term cardioprotective effects of the Traditional Chinese Medicine MLC901 against myocardial ischemia-reperfusion injury in mice." Sci Rep 7(1): 14701.
6. Galaup, A., E. Gomez, R. Souktani, M. Durand, A. Cazes, C. Monnot, J. Teillon, S. Le Jan, C. Bouleti, G. Briois, J. Philippe, S. Pons, V. Martin, R. Assaly, P. Bonnin, P. Ratajczak, A. Janin, G. Thurston, D. M. Valenzuela, A. J. Murphy, G. D. Yancopoulos, R. Tissier, A. Bordeaux, B. Ghaleh and S. Germain (2012). "Protection against myocardial infarction and no-reflow through preservation of vascular integrity by angiopoietin-like 4." Circulation 125(1): 140-149.

## Claims

1. Conjugate comprising a peptide having the amino acid sequence outlined in SEQ ID NO: 1 or SEQ ID NO: 2, linked to a cell-penetrating peptide (CPP), for use in the treatment of a condition due to microvascular injury.

2. Conjugate for use according to claim 1, wherein microvascular injury is microhemorrhage.

3. Conjugate for use according to claim 2, wherein microhemorrhage is a condition chosen among Intra Cerebral Hemorrhage (ICH), hypertension hemorrhage, sub-arachnoid hemorrhage (SARAH), hemorrhage due to retinopathy of prematurity (ROP), hemorrhage due to proliferative diabetic retinopathy (PDR), dengue hemorrhage fiver, intramyocardial bleeding after Acute myocardial infarction (AMI), intracranial hemorrhage after stroke, and hemorrhage after COVID-19 infection.

4. Conjugate for use according to claim 1, wherein said condition is a no-reflow phenomenon.

5. Conjugate for use according to any one of the preceding claims, wherein the CPP is selected from the group consisting of Tat, RXR, Bpep, and Pip2b.

6. Conjugate for use according to claim 5, wherein the CPP is Tat.

7. Conjugate for use according to claim 6, having the amino acid sequence set forth in SEQ ID NO: 4 or SEQ ID NO: 5.

8. Pharmaceutical composition comprising an effective amount of at least one conjugate as defined in claim 1, and at least one pharmaceutically acceptable carrier or excipient, for use in the treatment of a condition due to microvascular injury.

9. Pharmaceutical composition for use according to claim 8, wherein the CPP is Tat.

10. Pharmaceutical composition for use according to claim 9, having the amino acid sequence set forth in SEQ ID NO: 4 or SEQ ID NO: 5.
